# EUROPEAN PATENT APPLICATION

(11) **EP 2 028 276 A1**
(43) Date of publication of application: **25.02.2009**
(21) Application number: 07743545.1
(22) Date of filing: 17.05.2007
(51) Int. Cl.: C12P 13/02, A61K 31/164, A61P 17/00, C12R 1/01, C12R 1/02

(54) **METHOD FOR PRODUCTION OF ACETIC ACID BACTERIUM-TYPE CERAMIDE**

(30) Priority: 22.05.2006 JP 2006141323
(71) Applicant: Mizkan Group Corporation, Handa-shi, Aichi 475-8585 (JP)
(72) Inventor: OGAWA, Shin, Handa-shi, Aichi 475-8585 (JP); ODA, Takahiro, Handa-shi, Aichi 475-8585 (JP)
(74) Representative: Uchida, Kenji
(86) International application number: PCT/JP2007/060110
(87) International publication number: WO 2007/135941

(57) **Abstract**

[Problems] To provide a method for efficient production of an acetic acid bacterium-type ceramide which is expected to have physiological effects such as making skin beautiful, by increasing the content of ceramide contained in cells of an acetic acid bacterium.

[Means for Solving the Problems] There has been developed a method for increasing the acetic acid bacterium-type ceramide where the content of the acetic acid bacterium-type ceramide in cells of the acetic acid bacterium is able to be made as high as to a level of 10 to 30 fold by such a manner that acetic acid bacterium after finishing the culture is kept at the pH of 2.0 to 8.0 and the temperature of 4 to 70°C for 3 hours to 7 days. As to the acetic acid bacterium used, *Acetobacter malorum* NCI 1683 strain (FERM BP-10595) or *Gluconacetobacteri hansenii* NCI 1468 strain (FERM BP-10596) is preferred.

## Description

### Technical Field

The present invention relates to a method for the production of ceramide. More particularly, it relates to an effective method for the production of N-2'-hydroxypalmitoyl-sphinganine (hereinafter, it may be sometimes called "acetic acid bacterium-type ceramide") which is a kind of ceramide by means of increasing its content in cells of acetic acid bacterium.

### Background Art

Ceramide is a general name for the substances where fatty acid is amide-bonded to amino group of sphingosine which is a kind of sphingoid long-chain. Ceramide is a kind of sphingolipids, and is widely distributed among microbes and animals and plants. Particularly in humans, ceramide is characteristically present as a constituting component of skin organs and plays an important role such as prevention of loss of water and drying of skin.

It has been known already that, when ceramide derived from plants such as wheat, rice and soybean is orally ingested, the effects of making skin beautiful such as improvement of moisture retention, prevention of skin roughness and wrinkles is achieved (refer, for example, to Non-Patent Document 1 and Non-Patent Document 2) but there is a problem that the ceramide derived from plants is different in its chemical structure from the ceramide derived from animals such as human and its effect of making skin beautiful is weak.

On the other hand, ceramide extracted from animals such as bovine brain is similar in its chemical structure to human ceramide (hereinafter, it will be sometimes called "human-type ceramide") and, its ability of making skin beautiful is strong, however, there is a tendency that utilization of ceramide derived from animals such as bovine brain to human by means of oral ingestion or the like is avoided as a result of accidents by Bovine Spongiform Encephalopathy (BSE) or the like.

Although the ceramide derived from microbes is similar to human-type ceramide in its chemical structure and the high effect is expected, there is still a feeling of doubt for the safety in the case of ceramide derived from microbes such as *Sphingomonas* which have not been used for food yet, whereby its oral ingestion has been politely avoided.

On the other hand, an acetic acid bacterium which is a kind of prokaryote is a highly safe microbe which has been traditionally utilized for the manufacture of vinegar and has been confirmed to contain an acetic acid bacterium-type ceramide (refer, for example, to Non-Patent Document 3).

The acetic acid bacterium-type ceramide has a structure where fatty acid is amide-bonded to sphinganine which is a precursor of sphingosine being a sphingoid base part of ceramide derived from animals and, since it has a structure common to human-type ceramide, it has been expected to have a strong physiological activity such as an effect of making the human skin beautiful.

In view of the above, there has been a demand for a highly efficient production of an acetic acid bacterium-type ceramide having a high added-value by using a highly safe acetic acid bacterium which has been traditionally utilized for the manufacture of vinegar.

However, for example, in a representative acetic acid bacterium used for brewery of vinegar, the content of ceramide contained in the cells has been estimated to be about 2 to 4 mg (3.5 to 7 µmol) per gram of dried cells (refer, for example, to Non-Patent Document 3 and Non-Patent Document 4) and that is never satisfactory.

Furthermore, an acetic acid bacterium has a low growth ability and, therefore, the yield of the cells of an acetic acid bacterium is little, that is one of the reasons why the manufacturing efficiency of an acetic acid bacterium-type ceramide using an acetic acid bacterium is low.

With regard to an example for the improvement where the content of an acetic acid bacterium-type ceramide in the cells of an acetic acid bacterium is increased, there is a disclosure that, when an acetic acid bacterium is cultured in a medium to which ethanol is added, the content of ceramide in the cells increases to an extent of about twice as compared with the case where culture is done in a medium to which no ethanol is added (refer, for example, to Non-Patent Document 5). However, there has been a demand for developing a method where the content of an acetic acid bacterium-type ceramide in the cells of an acetic acid bacterium is still increased further, growth of the acetic acid bacterium is promoted, and thus the acetic acid bacterium-type ceramide is more efficiently produced.
Non-Patent Document 1: Fragrance Journal, Volume 23, pages 81 to 89, 1995
Non-Patent Document 2: Bioindustry, Volume 19, pages 16 to 26, 2002
Non-Patent Document 3: Obihiro Daigaku Kenkyu Hokoku, Volue 23, pages 917 to 925, 1978
Non-Patent Document 4: Doctoral Dissertation, United Agricultural Course of the Post-Graduated School of Iwate University, by Hidetsugu Goto, pages 11 to 41, 2001
Non-Patent Document 5: Shishitsu Seikagaku Kenkyu, Volume 42, pages 246 to 249, 2000

### Disclosure of the Invention

### Problems that the Invention is to Solve

An object of the present invention is to develop a method where the content of an acetic acid bacterium-type ceramide in the cells of an acetic acid bacterium is increased and to provide a method where the acetic acid bacterium-type ceramide which has been expected to have physiological effects such as making skin beautiful is able to be efficiently produced.

### Means for Solving the Problems

In view of the above problems, the present inventors have conducted intensive investigations and, as a result, the content of ceramide in the cells of an acetic acid bacterium significantly increases when the acetic acid bacterium is cultured, and thereafter, the collected cells of the acetic acid bacterium are treated with exposing to the state of a low pH and a high temperature has been found.
Moreover, the optimum environmental conditions during the said treatment have been determined, and the acetic acid bacterium used therefor and a culture condition therefor have been investigated whereupon the present invention has been accomplished.

Thus, the present invention relates to each of the following items.

(1) A method for increasing the content of an acetic acid bacterium-type ceramide in the cells of an acetic acid bacterium, characterized in that, the acetic acid bacterium after finishing the culture is kept for 3 hours to 7 days at the pH of 2.0 to 8.0 and/or at the temperature of 4 to 80°C.

(2) A method for increasing the content of an acetic acid bacterium-type ceramide in the cells of an acetic acid bacterium, characterized in that, the acetic acid bacterium after finishing the culture is kept for 1 day to 4 days at the pH of 2.0 to 4.5 and/or at the temperature of 30 to 70°C.

(3) The method for increasing the content of an acetic acid bacterium-type ceramide in the cells of an acetic acid bacterium according to the above (1) or (2), wherein the acetic acid bacterium cultured in a medium containing acetic acid and containing not more than 0. 3 volume/volume % of ethanol is used.

(4) The method for increasing the content of an acetic acid bacterium-type ceramide in the cells of an acetic acid bacterium according to any of the above (1) to (3), wherein *Acetobacter malorum* NCI 1683 strain (FERM BP-10595) is used as the acetic acid bacterium.

(5) The method for increasing the content of an acetic acid bacterium-type ceramide in the cells of an acetic acid bacterium according to any of the above (1) to (3), wherein *Gluconacetobacter hansenii* NCI 1468 strain (FERM BP-10596) is used as the acetic acid bacterium.

(6) The method for increasing the content of an acetic acid bacterium-type ceramide in the cells of an acetic acid bacterium according to any of the above (1) to (5), wherein the content of ceramide is not less than 6 mg, preferably not less than 7 mg, more preferably not less than 9 mg and, particularly preferably, not less than 11 mg and not more than 20 mg per gram of dry cells.

### Advantages of the Invention

In accordance with the present invention, it is now possible that the content of an acetic acid bacterium-type ceramide which has been expected to have a strong physiological action contained in the cells of an acetic acid bacterium is able to be increased and that the yield of the acetic acid bacterium cells is also able to be increased and accordingly that the acetic acid bacterium-type ceramide is able to be produced more efficiently than prior art.

### Best Mode for Carrying Out the Invention

The present invention will now be illustrated in detail as follows.

With regard to the acetic acid bacterium used in the present invention, there is no particular limitation so far as it is an acetic acid bacterium which produces ceramide and examples thereof are the acetic acid bacteria belonging to the genus *Gluconacetobacter,* the genus *Gluconobacter*, the genus *Acetobacter,* the genus *Asaia* and the genus *Acidomonas*.

To be more specific, examples of the acetic acid bacteria belonging to the genus *Gluconacetobacter* are *Gluconacetobacter hansenii, Gluconacetobacter diazotrophicus, Gluconacetobacter intermedius, Gluconacetobacter sacchari, Gluconacetobacter xylinus, Gluconacetobacter europaeus* and *Gluconacetobacter oboediens*, etc.

Examples of the acetic acid bacteria belonging to the genus *Gluconobacter* are *Gluconobacter frateurii* and *Gluconobacter cerinus*, etc.

Examples of the acetic acid bacteria belonging to the genus *Acetobacter* are *Acetobacter tropicalis, Acetobacter indonesiensis, Acetobacter syzygii, Acetobacter cibinongensis, Acetobacter orientalis, Acetobacter pasteurianus, Acetobacter orleanensis, Acetobacter lovaniensis, Acetobacter aceti, Acetobacter pomorum* and *Acetobacter malorum,* etc.

Examples of the acetic acid bacteria belonging to the genus *Asaia* are *Asaia bogorensis and Asaia siamensis*, etc.

Examples of the acetic acid bacteria belonging to the genus *Acidomonas* are *Acidomonas methanolicus*, etc.

As to the acetic acid bacteria, it is also possible to appropriately utilize an acetic acid bacterium used for the manufacture of vinegar, that isolated from nature and that deposited in the culture collection organizations and available to the public.

Among the above acetic acid bacteria, that belonging to *Acetobacter malorum* has a high growth ability, contain a high content of acetic acid bacterium-type ceramide therein and has a particularly excellent growth in a medium containing acetic acid which will be mentioned later. Therefore, it is suitable for the efficient production of acetic acid bacterium-type ceramide and, among said bacteria, *Acetobacter malorum* NCI 1683 strain (FERM BP-10595) is particularly advantageously used in the present invention.

Said *Acetobacter malorum* NCI 1683 strain (FERM BP-10595) is an acetic acid bacterium isolated from smetana which is a fermented food in Russia. Since it has a Q9-type ubiquinone and its sequence of 16SrRNA is entirely identical to that of *Acetobacter malorum,* it is a strain identified as *Acetobacter malorum* (refer, for example, to International Journal of Systematic and Evolutionary Microbiology, Volume 52, pages 1551 to 1558, 2002) and has been internationally deposited at the Incorporated Administrative Agency, International Patent Organism Depositary, National Institute of Advanced Industrial Science and Technology (AIST Tsukuba Central 6, 1-1, Higashi-1-chome, Tsukuba-shi, Ibaraki-ken, 305-8566, Japan Zip code: 305-8566) under an accession number FERM BP-10595 on April 7, 2006.

Among the above acetic acid bacteria, a bacterium belonging to *Gluconacetobacter hansenii* also has a high growth ability, contains a high content of acetic acid bacterium-type ceramide and has a particularly excellent growth in a medium containing acetic acid the same as in the case of *Acetobacter malorum.* Accordingly, it is also suitable for an efficient production of an acetic acid bacterium-type ceramide and, among the bacteria, *Gluconacetobacter hansenii* NCI 1468 strain (FERM BP-10596) is advantageously used in the present invention.

Said *Gluconacetobacter hansenii* NCI 1468 strain (FERM BP-10596) is an acetic acid bacterium separated from nata de coco. Since it has a Q10-type ubiquinone and its sequence of 16SrRNA is entirely identical to that of *Gluconacetobacter hansenii,* it is a strain identified as *Gluconacetobacter hansenii* and has been internationally deposited at the Incorporated Administrative Agency, International Patent Organism Depositary, National Institute of Advanced Industrial Science and Technology (AIST Tsukuba Central 6, 1-1, Higashi-1-chome, Tsukuba-shi, Ibaraki-ken, 305-8566, Japan Zip code: 305-8566) under an accession number FERM BP-10596 on April 7, 2006.

In the present invention, such an acetic acid bacterium is cultured and thereafter the cells are collected and subjected to a high-temperature treatment at low pH whereby it is now possible to significantly increase the content of the acetic acid bacterium-type ceramide contained in the acetic acid bacterium.

There is no particular limitation for the method of culturing the acetic acid bacterium but any of the conventionally conducted methods is able to be adopted.

Thus, as to a medium, that containing carbon sources, nitrogen sources, inorganic substances, etc. is used and, if minor nutrients demanded by an acetic acid bacterium for its growth are contained therein in an appropriate amount, either synthetic medium or natural medium may be used. Examples of carbon sources are various kinds of carbohydrates such as glucose and sucrose. As to nitrogen sources, natural nitrogen sources such as peptone and decomposed products of fermentative microbe cells may be used.

As to the pH of the medium, it is usually preferred to be within a range of pH 2.5 to 7 and more preferred to be within a range of pH 2.7 to 6.5. The pH is able to be adjusted by using various kinds of acids, various kinds of bases, buffer, etc.

It is also possible to culture an acetic acid bacterium in a medium where ethanol is added to the above-mentioned medium and the adding amount of ethanol is usually about 0.5 to 8 volume/volume %.

In the present invention, a medium where acetic acid for assimilation by an acetic acid bacterium is added to the above-mentioned medium is able to be used more preferably. In that case, with regard to the condition where the assimilation of acetic acid takes place, attention is to be paid so that ethanol is to be hardly contained in the said medium. For example, ethanol concentration is preferably not more than about 0.3 volume/volume % and, more preferably, not more than about 0.1 volume/volume %. Acetic acid is preferred to be added in a form of an acetate and, although the concentration of the acetic acid (acetate) may be appropriately selected depending upon the acetic acid bacterium used, about 0.5 to 8 weight/volume % is selected.

As to a culturing method for an acetic acid bacterium, that which has been conventionally used for the culture of an acetic acid bacterium such as a static culture, a shaking culture and an aeration-agitation culture is acceptable and it is usually recommended that an acetic acid bacterium is cultured under an aerobic condition and at a temperature of about 30°C.

There is no particular limitation for a method where acetic acid bacterium cells are collected from the culture broth after finishing the incubation but a conventionally conducted centrifugal separation method and a concentrating method using a filter membrane may be exemplified.

In the present invention, the acetic acid bacterium recovered by collecting from the culture broth which is cultured by the above method is suspended in a solution and treated at a high-temperature and at low pH whereupon the content of ceramide in the acetic acid bacterium is able to be significantly increased.

As to a solution for suspending the acetic acid bacterium, anything such as water, buffer and culture liquid is able to be utilized. It is preferred that the pH of the solution for suspending the acetic acid bacterium is adjusted to 2.0 to 8.0 since the content of the acetic acid bacterium-type ceramide in the acetic acid bacterium is able to be more significantly increased by doing so. It is not preferred to make the pH lower than 2.0, since an effect of increasing the content of the acetic acid bacterium-type ceramide contained in the cells of the acetic acid bacterium becomes weak. On the other hand, if the pH is made higher than 8, the outcome is also the same and that is not preferred as well. More preferred pH is 2.0 to 4.5. Particularly preferred pH is 2 to 3. In order to adjust the pH of the solution in which the acetic acid bacterium is suspended to such an extent, various kinds of acids and salts which are able to lower the pH may be used and examples thereof are an organic acid such as acetic acid and citric acid, an inorganic acid such as hydrochloric acid and sulfuric acid and salts thereof.

There is no particular limitation for a treating method and an example thereof is a method where neither shaking nor aeration-agitation is conducted but just by being allowed to be kept in static state. Period for the treatment is preferred to be 3 hours to 7 days. When the treating period is shorter than 1 day, an effect of increasing the content of the acetic acid bacterium-type ceramide contained in the cells of the acetic acid bacterium becomes weak and that is not preferred. When the treating period is longer than 7 days, the outcome is the same and that is not preferred. More preferably, it is 1 day to 4 days.

There is no particular limitation for the temperature for the treating so far as it is the temperature by which an effect of increasing the content of the acetic acid bacterium-type ceramide contained therein is achieved. An example thereof is a temperature range of from 4°C to 80°C. As to the preferred temperature range, that from 4°C to 70°C may be exemplified. At the temperature of lower than 30°C within the range as such, an effect of increasing the content of the acetic acid bacterium-type ceramide contained therein becomes weak and that is not preferred, while, when the temperature is as high as not lower than 80°C, the amount of the bacterium cells decreases and that is not preferred. Accordingly, the temperature range is preferably from 30°C to 80°C, more preferably from 40°C to 70°C and, particularly preferably, from 60°C to 70°C.

After the acetic acid bacterium where the content of the acetic acid bacterium-type ceramide contained therein is increased by the static treatment as above is subjected to a treatment such as washing and drying upon necessity, the bacterium is able to be utilized in various ways just as it is or after subjecting to a processing treatment such as a crushing treatment.

It is also possible that the acetic acid bacterium-type ceramide is utilized after extracting and purifying from the above acetic acid bacterium if necessary. As to the method for the extraction and the purification as above, any of the known methods for the extraction of lipid may be conducted and there is no particular limitation therefor.

An example thereof is a method where cells of the acetic acid bacterium obtained by the above treatment are collected and, after that, the acetic acid bacterium-type ceramide is extracted therefrom using a polar solvent such as ethanol, acetone, ethyl acetate and recrystallized in the organic solvent.

Examples of a method for quantitative determination of ceramide in the preset invention are a method where a lipid extract is made to react in the presence of pyridine and benzoyl chloride so that a benzoyl group is introduced thereinto followed by detecting by a high-performance liquid chromatography at the ultraviolet wavelength of 230 nm and a method where the lipid extract is detected using a light scattering detector by a high-performance liquid chromatography.

In accordance with the present invention, the content of ceramide contained in the cells is able to be greatly increased and it has been proved that 6 to 18.59 mg per gram of the dried cells of acetic acid bacterium is able to be achieved. To be more specific, the cases where not less than 7 mg, not less than 9 mg, not less than 11 mg, not less than 13 mg, not less than 14 mg, not less than 15 mg and not less than 18 mg have been actually achieved and, with regard to its upper limit, the case where it is not less than 19 mg or, for example, it is 20 mg is able to be well expected.

The acetic acid bacterium-type ceramide prepared as such is able to be utilized as a composition where a potent physiological action such as improvement in the skin function is able to be expected.

### Examples

The present invention will now be illustrated in more detail by way of the following Examples and Test Examples.

### (Example 1) Increasing the content of ceramide in the cells by means of a treatment at low pH

*Acetobacter malorum* NCI 1683 strain (FERM BP-10595) was inoculated to 5 ml of a YPG liquid medium (3 weight/volume % of glucose, 0.3 weight/ volume % of polypeptone and 0.5 weight/volume % of yeast extract; pH 6.5) and subjected to a shaking culture at 30°C for 48 hours at 120 rpm whereupon a pre-preculture was conducted.

After that, the pre-precultured broth was inoculated, in an amount of 2%, to 5 ml of a YPG liquid medium and subjected to a shaking culture at 30°C for 24 hours at 120 rpm whereupon a preculture was conducted.

The prepared above precultured liquid was inoculated, in an amount of 1%, to a two-liter jar fermenter containing a medium prepared by addition of sodium acetate to 1 liter of a YPG liquid medium so as to make the final concentration of sodium acetate 0.8 weight/volume % (prepared in such a manner that a 20 weight/volume % aqueous solution of sodium acetate adjusted to pH 6.5 with 1N hydrochloric acid is added so as to make the final concentration of sodium acetate 0.8 weight/volume %) and subjected to an aeration-agitation culture at 28°C for 48 hours at 500 rpm where aeration of 0.5 liter/minute was conducted whereupon 1 liter of a culture liquid after completion of the culture was prepared. The resulting culture broth was centrifuged at 8,000 g for 5 minutes and the recovered acetic acid bacteria cells were re-suspended in a 1/6 volume of a culture supernatant liquid (pH 4.2) to prepare a 6-fold concentrated cell suspension.

Each 5 ml of the 6-fold concentrated cell suspension was dispensed in a 15-ml Falcon tube ( Trade name of centrifuge tube ) and 6N hydrochloric acid or 6N sodium hydroxide solution was added so as to adjust each of the final pH from 2 to 8. The pH of the test section where the above pH adjustment was not conducted was 4.2. Those Falcon tubes were allowed to be kept in static state for 4 days under warming at 40°C.

After that, the cell suspension was centrifuged to collect the cells and the cells were washed by suspending in 10 ml deionized water again, and by collecting again, and then were freeze-dried.

After finishing the freeze-drying, amount (in grams) of the acetic acid bacterium cells was measured.

Each 10 mg of the acetic acid bacterium cells prepared by the freeze-drying was extracted with an organic solvent composition comprising chloroform, methanol and water (1:2:0.8) according to the known Bligh-dyer method so that the total lipid was extracted.

After that, the organic solvent was evaporated from the total lipid followed by concentrating to dryness, the residue was subjected to a weak alkaline hydration by addition of 0.4N potassium hydroxide solution thereto and the recovered alkaline stable lipids were made to react in the presence of anhydrous pyridine and benzoyl chloride at 70°C for 10 minutes to purify benzoyl derivatives containing sphingolipid and hopanoid.

The benzoyl derivatives were subjected to a high-performance liquid chromatography to detect the absorbance of ultraviolet at wavelength of 230 nm. At that time, an acetic acid bacterium-type ceramide was quantified by using a standard curve prepared by using purified N-2'-hydroxypalmitoyl- sphinganine (an acetic acid bacterium-type ceramide) extracted from *Gluconacetobacter xylinus* NBRC 15237 strain.

Based on the resulting quantified value of the acetic acid bacterium-type ceramide, the content of the acetic acid bacterium-type ceramide per 1 g of the dried acetic acid bacterium was calculated and the effect of the treatment was compared.

A mobile phase of the high-performance liquid chromatography was consist of hexane and isopropanol (100:1), and was pumped at a flow rate of 1 ml/minute.

Table 1 shows the result of determination of the amount of dried acetic acid bacterium cells (g/liter) per 1 liter of the medium, the content of the acetic acid bacterium-type ceramide (mg/g) per 1 g of the dried acetic acid bacterium cells and the amount of the produced acetic acid bacterium-type ceramide (mg/liter) per 1 liter of the medium. (Table 1: Effect of pH)

Incidentally, the above static treatment was not conducted but an immediate freeze-drying was conducted and then the amount of dried acetic acid bacterium cells (g/liter) per 1 liter of the medium, the content of the acetic acid bacterium-type ceramide (mg/g) per 1 g of the dried acetic acid bacterium cells and the amount of the produced acetic acid bacterium-type ceramide (mg/liter) per 1 liter of the medium were measured in the same manner as mentioned above and the result was defined as "before the treatment".

**[Table 1]**

| Treating Condition | Amount of Dried Acetic Acid Bacterium Cells | Content of the Ceramide | Amount of the Produced Ceramide |
|---|---|---|---|
| Before the Treatment | 1.90 | 0.40 | 0.76 |
| pH 8.0 | 1.57 | 1.72 | 2.7 |
| pH 7.0 | 1.58 | 2.45 | 3.9 |
| pH 6.0 | 1.58 | 3.28 | 5.2 |
| pH 5.0 | 1.63 | 4.26 | 6.9 |
| pH 4.2 | 1.60 | 7.63 | 12.2 |
| pH 3.0 | 1.64 | 13.17 | 21.6 |
| pH 2.5 | 1.48 | 15.08 | 22.3 |
| pH 2.0 | 1.38 | 13.18 | 18.2 |

It is apparent from the result of Table 1 that, even when the pH of the acetic acid bacterium cell suspension was adjusted to any of the values of 2.0 to 8.0, the content of the ceramide therein increased to an extent of not less than 4-fold as compared with the case of before the treatment and the content of the ceramide produced thereby also increased accordingly.

It was confirmed that, when the pH was lowered to 4.5 or less, the content of the ceramide contained therein increased to an extent of 4-fold or more as compared with the case of pH 8.0 and that the content of the ceramide produced thereby also significantly increased accordingly.

It was able to be confirmed from the above result that the pH is preferably from 2.0 to 8.0, more preferably from 2.0 to 4.5 and, particularly preferably, from 2.0 to 3.0.

### (Example 2) Effect of acetic acid

The same as in Example 1, *Acetobacter malorum* NCI 1683 strain (FERM BP-10595) was cultured for 48 hours in a medium where sodium acetate was added to a YPG medium so as to make its final concentration 0.8%, the resulting culture broth was centrifuged at 8,000 g for 5 minutes and the recovered acetic acid bacterium cells were re-suspended in a 1/6-fold volume of a supernatant culture liquid to prepare a 6-fold concentrated cell suspension.

After that, 99.5% acetic acid was added thereto so as to make its final concentration 5% and the cell suspension was allowed to be kept in static state at 40°C for 4 days. When acetic acid was added to make the final concentration 5%, pH of the concentrated cell suspension became 3.5.

The cells were collected, washed and freeze-dried in the same manner as in Example 1, then the amount of dried acetic acid bacterium cells (g/liter) per 1 liter of the medium, the content of the acetic acid bacterium-type ceramide (mg/g) per 1 g of the dried acetic acid bacterium cells and the amount of the produced acetic acid bacterium-type ceramide (mg/liter) per 1 liter of the medium were measured and the result is shown in Table 2. (Table 2: Effect of Acetic Acid)
Incidentally, the above keeping in static state was not conducted but an immediate freeze-drying was conducted and then the amount of dried acetic acid bacterium cells (g/liter) per 1 liter of the medium, the content of the acetic acid bacterium-type ceramide (mg/g) per 1 g of the dried acetic acid bacterium cells and the amount of the produced acetic acid bacterium-type ceramide (mg/liter) per 1 liter of the medium were measured in the same manner as mentioned in Example 1 and the result was defined as "before the treatment".

**[Table 2]**

| Test Sections | Amount of Dried Acetic Acid Bacterium Cells | Content of the Ceramide | Amount of the Produced Ceramide |
|---|---|---|---|
| Before the Treatment | 1.90 | 0.4 | 0.76 |
| After the Treatment | 1.55 | 10.16 | 15.8 |

It was able to be confirmed from the result of Table 2 that, when pH was lowered by addition of acetic acid, content of ceramide significantly increased to an extent of 25-fold as compared with the case of before the treatment. It was therefore confirmed that the acid which is possible to be used for lowering the pH for increasing the content of ceramide is not limited to hydrochloric acid only.

### (Example 3) Increasing the content of ceramide by a high-temperature treatment

In the same manner as in Example 1, *Acetobacter malorum* NCI 1683 strain (FERM BP-10595) was cultured for 48 hours in a medium where sodium acetate was added to a YPG medium so as to make its final concentration 0.8%, the resulting culture broth was centrifuged at 8, 000 g for 5 minutes and the recovered acetic acid bacterium cells were re-suspended in a 1/6-fold volume of a supernatant culture liquid to prepare a 6-fold concentrated cell suspension.

After that, the above 6-fold concentrated cell suspension was adjusted to pH 3 by 6N hydrochloric acid in the same manner as in Example 2 and allowed to be kept in static state for 4 days in an incubator of the temperature as shown in Table 3.

In accordance with the method mentioned in Example 1, the sample after the keeping at high-temperature was subjected to collection of the cells followed by washing, the resulting cells were freeze-dried, the amount of dried acetic acid bacterium cells (g/liter) per 1 liter of the medium, the content of the acetic acid bacterium-type ceramide (mg/g) per 1 g of the dried acetic acid bacterium cells and the amount of the produced acetic acid bacterium-type ceramide (mg/liter) per 1 liter of the medium were measured and the result is shown in Table 3. (Table 3: Effect of temperature)

Incidentally, the above treatment by being allowed to be kept in static state was not conducted but an immediate freeze-drying was conducted and then the amount of dried acetic acid bacterium cells (g/liter) per 1 liter of the medium, the content of the acetic acid bacterium-type ceramide (mg/g) per 1 g of the dried acetic acid bacterium cells and the amount of the produced acetic acid bacterium-type ceramide (mg/liter) per 1 liter of the medium were measured in the same manner as mentioned in Example 1 and the result was defined as "before the treatment".

**[Table 3]**

| Temperature | Amount of Dried Acetic Acid Bacterium Cells | Content of the Ceramide | Amount of the Produced Ceramide |
|---|---|---|---|
| Before the Treatment | 1.90 | 0.40 | 0.76 |
| 4°C | 1.84 | 1.23 | 2.3 |
| 10°C | 1.91 | 1.43 | 2.7 |
| 20°C | 1.78 | 4.06 | 7.2 |
| 30°C | 1.73 | 9.43 | 16.3 |
| 37°C | 1.67 | 11.70 | 19.5 |
| 40°C | 1.64 | 13.17 | 21.6 |
| 45°C | 1.64 | 13.90 | 22.7 |
| 50°C | 1.58 | 14.53 | 23.0 |
| 60°C | 1.42 | 18.35 | 26.1 |
| 70°C | 1.28 | 18.59 | 23.8 |

It is apparent from the result of Table 3 that, when the temperature of the acetic acid bacterium cell suspension was adjusted to any of 4°C to 70°C, the content of the ceramide contained therein increased to an extent of not less than 3-fold as compared with the case of before the treatment and the content of produced ceramide also increased accordingly.

It was also confirmed that, when the temperature was adjusted to 30°C or higher, the content of the ceramide increased to an extent of 8-fold or more as compared with the case where the temperature was 4°C and the amount of the produced ceramide also significantly increased accordingly.

From the above result, the preferred temperature was found to be 30 to 70°C and, when it is 60 to 70°C, the content of the ceramide increased up to 18 mg or even more.

### (Example 4) Effect of the treating time

In the same manner as in Test Example 1, *Acetobacter malorum* NCI 1683 strain (FERM BP-10595) was cultured for 48 hours in a medium where sodium acetate was added to a YPG medium so as to make its final concentration 0.8%, the resulting culture broth was centrifuged at 8, 000 g for 5 minutes and the collected acetic acid bacterium cells were re-suspended in a 1/6-fold volume of a supernatant culture liquid to prepare a 6-fold concentrated cell suspension.
After that, the above 6-fold concentrated cell suspension was adjusted to pH 3 by 6N hydrochloric acid in the same manner as in Test Example 2 and allowed to be kept in static state in an incubator at 40°C during the time as shown in Table 4.

In accordance with the method mentioned in Example 1, the sample after the high-temperature treatment was subjected to collection of the cells followed by washing and the resulting cells were freeze-dried. After that, the cells were collected, washed and freeze-dried in the same manner as in Example 1, then the amount of dried acetic acid bacterium cells (g/liter) per 1 liter of the medium, the content of the acetic acid bacterium-type ceramide (mg/g) per 1 g of the dried acetic acid bacterium cells and the amount of the produced acetic acid bacterium-type ceramide (mg/liter) per 1 liter of the medium were measured and the result is shown in Table 4. (Table 4: Effect of the treating time)

Incidentally, the above treatment by being allowed to be kept in static state was not conducted but an immediate freeze-drying was conducted and then the amount of dried acetic acid bacterium cells (g/liter) per 1 liter of the medium, the content of the acetic acid bacterium-type ceramide (mg/g) per 1 g of the dried acetic acid bacterium cells and the amount of the produced acetic acid bacterium-type ceramide (mg/liter) per 1 liter of the medium were measured in the same manner as mentioned in Example 1 and the result was defined as "before the treatment".

**[Table 4]**

| Treating Time | Amount of Dried Acetic Acid Bacterium Cells | Content of the Ceramide | Amount of the Produced Ceramide |
|---|---|---|---|
| Before the Treatment | 1.90 | 0.40 | 0.76 |
| 3 hours | 1.85 | 1.20 | 2.2 |
| 6 hours | 1.81 | 2.03 | 3.6 |
| 12 hours | 1.77 | 3.51 | 6.2 |
| 1 day | 1.64 | 7.70 | 12.6 |
| 3 days | 1.72 | 9.54 | 16.4 |
| 4 days | 1.64 | 13.17 | 21.6 |
| 5 days | 1.89 | 11.68 | 22.1 |
| 7 days | 1.84 | 11.83 | 21.8 |
| 10 days | 1.70 | 9.43 | 16.0 |
| 17 days | 1.52 | 13.77 | 20.9 |
| 22 days | 1.51 | 15.28 | 23.0 |

It was able to be confirmed from the result of Table 4 that, when the treating time was made 3 hours or longer, the content of the ceramide contained in the acetic acid bacterium increased to an extent of not less than 3-fold as compared with the case of before the treatment and the amount of the produced ceramide also increased accordingly. It was also able to be confirmed that, when the treatment was conducted for 1 day or longer, the content of ceramide contained therein increased to an extent of 6-fold or more and the amount of the produced ceramide also increased accordingly as compared with the case where the treatment was conducted for 3 hours.

On the other hand, even when the treatment was conducted for 7 days or longer, no significant increase in the content of the ceramide was able to be confirmed.

It was confirmed from the above result that, as to the treating time, 3 hours to 7 days is preferred and 1 day to 4 days is more preferred.

### (Example 5) Ceramide production test in the strain belonging to the genus Gluconacetobacter

In the same manner as in Example 1, *Gluconacetobacter hansenii* NCI 1468 strain (FERM BP-10596) was cultured for 48 hours in a medium where sodium acetate was added to a YPG medium so as to make its final concentration 0.8%, the resulting culture broth was centrifuged at 8,000 g for 5 minutes and the recovered acetic acid bacterium cells were re-suspended in a 1/6-fold volume of a supernatant culture liquid to prepare a 6-fold concentrated cell suspension.

After that, 99.5% acetic acid was used and added so as to make its final concentration 5% followed by being allowed to be kept in static state at 37°C for 2 days.

In accordance with the method mentioned in Example 1, the sample after being allowed to be kept in static state was subjected to collection of the cells followed by washing, the resulting cells were freeze-dried, then the amount of dried acetic acid bacterium cells (g/liter) per 1 liter of the medium, the content of the acetic acid bacterium-type ceramide (mg/g) per 1 g of the dried acetic acid bacterium cells and the amount of the produced acetic acid bacterium-type ceramide (mg/liter) per 1 liter of the medium were measured and the result as compared with the case of before the treatment is shown in Table 5. (Table 5: Ceramide production test in the genus belonging to *Gluconacetobacter*)

**[Table 5]**

| Test Sections | Amount of Acetic Acid Bacterium Cells | Content of the Ceramide | Amount of the Produced Ceramide |
|---|---|---|---|
| Before the Treatment | 2.11 | 0.42 | 0.9 |
| After the Treatment | 1.88 | 3.30 | 6.2 |

It was able to be confirmed from the result of Table 5 that the content of ceramide increased not only in the acetic acid bacterium belonging to the genus *Acetobacter* but also in the acetic acid bacterium belonging to the genus *Gluconacetobacter* by the method of the present invention and, as a result thereof, the amount of the produced ceramide significantly increased as well. It was confirmed in view of the above that the method of the present invention is able to be used in any of acetic acid bacterium.

## Claims

1. A method for increasing the content of an acetic acid bacterium-type ceramide in the cells of an acetic acid bacterium, **characterized in that,** the acetic acid bacterium after finishing the culture is kept for 3 hours to 7 days at the pH of 2.0 to 8.0 and/or at the temperature of 4 to 80°C.

2. A method for increasing the content of an acetic acid bacterium-type ceramide in the cells of an acetic acid bacterium, **characterized in that,** the acetic acid bacterium after finishing the culture is kept for 1 day to 4 days at the pH of 2.0 to 4.5 and/or at the temperature of 30 to 70°C.

3. The method for increasing the content of an acetic acid bacterium-type ceramide in the cells of an acetic acid bacterium according to the above (1) or (2), wherein the acetic acid bacterium cultured in a medium containing acetic acid and containing not more than 0. 3 volume/volume % of ethanol is used.

4. The method for increasing the content of an acetic acid bacterium-type ceramide in the cells of an acetic acid bacterium according to any of the above (1) to (3), wherein *Acetobacter malorum* NCI 1683 strain (FERM BP-10595) is used as the acetic acid bacterium.

5. The method for increasing the content of an acetic acid bacterium-type ceramide in the cells of an acetic acid bacterium according to any of the above (1) to (3), wherein *Gluconacetobacter hansenii* NCI 1468 strain (FERM BP-10596) is used as the acetic acid bacterium.

6. The method for increasing the content of an acetic acid bacterium-type ceramide in the cells of an acetic acid bacterium according to any of the above (1) to (5), wherein the content of ceramide is not less than 6 mg, preferably not less than 7 mg, more preferably not less than 9 mg and, particularly preferably, not less than 11 mg and not more than 20 mg per gram of dry cells.
